# EUROPEAN PATENT APPLICATION

(11) **EP 3 257 940 A1**
(43) Date of publication of application: **20.12.2017**
(21) Application number: 16749175.2
(22) Date of filing: 05.02.2016
(51) Int. Cl.: C12N 15/09, A61K 31/711, A61K 31/713, A61P 35/00, A61P 35/04, C12N 15/115, C12Q 1/68

(54) **DNA APTAMER CAPABLE OF BINDING TO NON-SMALL CELL LUNG CANCER CELL (H1975)**

(30) Priority: 10.02.2015 JP 2015024165
(71) Applicant: Nissan Chemical Industries, Ltd., Chiyoda-ku Tokyo 101-0054 (JP)
(72) Inventor: FURUSHO, Hitoshi, Funabashi-shi Chiba 274-0052 (JP)
(74) Representative: J A Kemp
(86) International application number: PCT/JP2016/053560
(87) International publication number: WO 2016/129531

(57) **Abstract**

The objective of the present invention is to provide a novel DNA aptamer useful for the diagnosis, treatment, and prevention of metastasis, and the like, of lung cancer.

A DNA aptamer comprising specifically binding to a non-small cell lung cancer cell, in which the DNA aptamer has at least one nucleotide sequence selected from the sequence of SEQ ID NOs: 1 and 2; a composition for detecting lung cancer cell, comprising the DNA aptamer; a kit for detecting lung cancer cell, comprising the DNA aptamer; a method for detecting lung cancer comprising using the DNA aptamer; a detection method comprising contacting the DNA aptamer with a sample obtained from the living body selected from the group consisting of lung cells, lung tissues, blood, serum, plasma, saliva, and sputum and detecting the presence of a lung cancer cell by observing the response caused by the binding of the sample and the DNA aptamer; a pharmaceutical composition for preventing metastasis of or treating lung cancer, comprising the DNA aptamer, and a drug delivery system for preventing metastasis of or treating lung cancer, comprising the DNA aptamer.

## Description

### TECHNICAL FIELD

The present invention is related to a DNA aptamer that can specifically binding to cancer cells, in particular, non-small cell lung cancer cells (H1975) and a composition comprising the DNA aptamer.

### BACKGROUND ART

DNA is a biopolymer consisting of four nucleobases of guanine (G), cytosine (C), adenine (A), and thymine (T), and in a living body, it mainly takes a role of conservation, expression, and transmission of genes. However, in the recent years, the presence of the DNA specifically binding to the target substance has been revealed and studies on utilizing DNA *per se* as functional polymer has been promoted. So far, nucleotide sequences having high binding affinity ability to low molecular compounds such as pharmaceutical agents, DNAs, RNAs, peptides, proteins have been discovered and selected, and a DNA having a selective recognition ability to a specific molecule is referred to as a "DNA aptamer".

In general, the selection of nucleic acid aptamers such as DNA aptamers, is carried out by using a method based on combinatorial chemistry called as *in vitro* selection method, in particular, Systematic Evolution of Ligands by Exponential enrichment: SELEX method (For example, Non-Patent Documents 1 and 2; Patent Document 1). The SELEX method includes the multiple repetition of selection of a nucleic acid ligand (aptamer) which binds to a target substance and exponential amplification by PCR results in obtaining a nucleic acid molecule (a single-stranded DNA and RNA) having an affinity to a target substance. Moreover, in the recent years, various improvements have been made, and reports are made on such as methods with superior efficiency and selectiveness which can collect aptamers with lesser number of cycles and methods obtaining aptamers binding not only to low molecules and proteins but also to cells and tissues (more precisely, to the molecules present on the surface) (Cell-SELEX method; for example, Non-Patent Document 3).

Such DNA aptamers are similar to antibodies in the point that they are biopolymers having a molecule recognition ability. Nonetheless, they have an advantage over the antibodies in the following points: Easy synthesis and modification; Superior stability to environmental change such as heat and pH; Non-limiting target substance which serves as a subject, theoretically, an aptamer to any substance can be obtained, and superior cost performance due to the rapid and cheap amplification carried out by methods of PCR and the like.

On the other hand, the death rate from lung cancer in Japan has demonstrated a steadily increase since 1950, and from 1993 onwards, the number of lung cancer death has ranked first of the number of cancer death. Lung cancer is mainly divided into two types such as non-small cell lung cancer and small cell lung cancer, and the former accounts for 80 to 85% of total lung cancers. Depending on its tissue type, non-small cell lung cancer is classified into such as squamous cell carcinoma, adenocarcinoma, and large cell carcinoma, and 2/3 of them are unresectable cases already at the time of finding, and become the main part of therapy. Improving the treatment outcome of advanced lung cancer, i.e., improving the chemotherapy outcome is indispensable for the treatment outcome of non-small cell lung cancer. For this purpose, it is necessary to distinguish the normal cells and cancer cells, and a biopolymer specifically binding to cancer cells is required. Accordingly, development of a novel lung cancer biomarker useful for the development of early diagnosis and an effective therapeutic agent are demanded.

### PRIOR ART DOCUMENTS

### PATENT DOCUMENTS

Patent Document 1: WO/9119813

### NON-PATENT DOCUMENTS

Non-Patent Document 1: Lee,et al., Curr Opin Chem Biol., 10(3):282,2006
Non-Patent Document 2: Gilbert,et al., Circulation., 116(23):2678,2007
Non-Patent Document 3: Guo, et al., Int.J.Mol.Sci., 9(4):668,2008

### SUMMARY OF INVENTION

### Technical Problem

Based on such background, the present invention aims to provide a novel DNA aptamer useful for studies on diagnosis, treatment, prevention of metastasis of lung cancer as well as basic research in cancer such as cancer metastasis.

### Solution to Problem

The present inventors have carried out dedicated research to solve the above-mentioned problem, thereby a nucleotide sequence having a specific binding ability to non-small cell lung cancer cells has been specified by using Cell-SELEX methods. Accordingly, the DNA having the specific sequence has been newly found to be able to function as a specific aptamer to non-small cell lung cancer cells, and thus, the present invention has been completed.

That is the say to that in one embodiment the present invention is as follows:
(1) A DNA aptamer having at least one nucleotide sequence selected from the sequence of SEQ ID NOs: 1 and 2 and characterized by specifically binding to a non-small cell lung cancer cell;
(2) A DNA aptamer having a sequence comprising substitutions, deletions, or additions of 1 to 3 nucleotides in at least one nucleotide sequence selected from the sequence of SEQ ID NOs: 1 and 2, and characterized by specifically binding to a non-small cell lung cancer cell;
(3) A DNA aptamer specifically binding to a non-small cell lung cancer cell, and having a nucleotide sequence of 5'-P₁-X-P₂-3';
   wherein X is 1) a nucleotide sequence selected from the sequence of SEQ ID NOs: 1 and 2, or 2) a sequence comprising substitutions, deletions, or additions of 1 to 3 nucleotides, in a nucleotide sequence selected from the sequence of SEQ ID NOs: 1 and 2, and
   P₁ and P₂ are a first and second primer recognition sequence introduced for PCR amplification;
(4) The DNA aptamer according to the above-mentioned (3), wherein P₁ is a first primer recognition sequence of SEQ ID NO: 3 and P₂ is a second primer recognition sequence of SEQ ID NO: 4;
(5) The DNA aptamer according to any one of the above-mentioned (1) to (4), wherein the non-small cell lung cancer cell is a H1975 cell;
(6) The DNA aptamer according to any one of the above-mentioned (1) to (5), comprising at least one chemical modification selected from the group consisting of chemical substitution at the sugar chain moiety, chemical substitution at the phosphate ester moiety, and a chemical substitution at the nucleic acid base moiety;
(7) The DNA aptamer according to any one of the above-mentioned (1) to (6), having a fluorescent label at the 5' end or 3' end, and
(8) The DNA aptamer according to the above-mentioned (7), wherein the fluorescent label is 6-carboxy tetramethyl rhodamine, fluorescein isothiocyanate, 6-carboxyfluorescein-aminohexyl, or a cyanine family fluorescent dye.
   In another embodiment, the present invention is related to the detection of lung cancer cells by the above-mentioned DNA aptamer, and more specifically, the following is provided:
(9) A composition for detecting lung cancer cell, comprising the DNA aptamer according to any one of the above-mentioned (1) to (8);
(10) A kit for detecting lung cancer cell, comprising the DNA aptamer according to any one of the above-mentioned (1) to (8);
(11) A method for detecting lung cancer characterized by using the DNA aptamer according to any one of the above-mentioned (1) to (8);
(12) The method according the above-mentioned (11) comprising a step of contacting the DNA aptamer with a sample obtained from a living body selected from the group consisting of lung cells, lung tissues, blood, serum, plasma, saliva, and sputum and a step of detecting the presence of a lung cancer cell by observing the response caused by the binding of the sample and the DNA aptamer, and
(13) The detection method according to the above-mentioned (12), wherein the response is a fluorescent response or a Raman scattering response.
   In a further embodiment, the present invention is related to using the above-mentioned DNA aptamer in a pharmaceutical composition and drug delivery system, and more specifically, the following is provided:
(14) A pharmaceutical composition for preventing metastasis of or treating lung cancer, comprising the DNA aptamer according to any one of the above-mentioned (1) to (8);
(15) A use of the DNA aptamer according to any one of the above-mentioned (1) to (8) for the production of a pharmaceutical composition for preventing metastasis of or treating lung cancer, and
(16) A drug delivery system for preventing metastasis of or treating lung cancer, comprising the DNA aptamer according to any one of the above-mentioned (1) to (8).

### ADVANTAGEOUS EFFECTS OF THE INVENTION

In accordance to the present invention, an efficient detection of a non-small cell lung cancer cell is made possible by using a novel DNA aptamer which specifically binds to the non-small cell lung cancer cell. In particular, the application to a kit comprising a DNA aptamer provided with a detection site such as fluorescent labelling and the like allows a convenient high-throughput detection and imaging with lung cells and tissues obtained from the living body as the subject of the measurement to be carried out. The detection of such lung cancer cells allows the diagnosis of development of cancer, the diagnosis of the presence of metastasis, the prognosis of cancer, and the diagnosis of severity.

Furthermore, the DNA aptamer of the present invention has a property that it could specifically bind to a non-small cell lung cancer cell, and then, the use of the above-mentioned DNA aptamer conjugated to a drug such as an anti-cancer agent and the like would allow the pharmaceutical agent to act certainly with the target site. Therefore, the DNA aptamer could be expected to be useful as a pharmaceutical composition for the prevention of metastasis or treatment of lung cancer or as a drug delivery system for such pharmaceutical composition.

Furthermore, as the consensus sequence in the DNA aptamer of the present invention is a relatively short region of only approximately 30 bases, it has an advantage that the time and trouble as well as cost for the production could be reduced, and the addition of a desired chemical modification and an additional function depending on the various uses could be easily carried out.

### BRIEF DESCRIPTION OF THE DRAWINGS

FIG.1 is a schematic diagram showing the making of the double-stranded DNA into a single strand using magnetic particles.
FIG. 2 is a fluorescent imaging figure of H1975 cells with a DNA aptamer having SEQ ID NO: 1.
FIG. 3 is a fluorescent imaging figure of H1975 cells with a DNA aptamer having SEQ ID NO: 2.

### DESCRIPTION OF EMBODIMENTS

Hereinafter, the embodiments of the present invention are described. Nonetheless, the scope of the present invention would not be restricted to these descriptions, and those other than the exemplifications mentioned below can be performed with appropriate modifications which are made without departing from the spirit of the present invention.

### 1. DNA aptamers

In the present application, a "DNA aptamer" refers to a single-stranded oligo DNA which can specifically recognize the target molecule or substance, and the DNA aptamer of the present invention is a single-stranded oligo DNA having a function specifically binding to a non-small cell lung cancer cell.

In general, a non-small cell lung cancer is mainly classified into squamous cell carcinoma (ASC), adenocarcinoma (ADC), and large cell carcinoma (LCC), depending on its tissue type. Accordingly, a non-limiting example of the "non-small cell lung cancer cell" of the present specification includes NCI-H226, NCI-H647 which are squamous cell carcinoma cells, NCI-H1975, A549, LC319, PC-3, PC-9, PC-14, A427, NCI-H1373 which are lung adenocarcinoma cells, and LX1 which are lung large cell carcinoma cells. The binding target of the DNA aptamer of the present invention is preferably adenocarcinoma cells, more preferably, H1975 cells (the details of H1975 cells are given in the following: Title of Research: The effect of opioids on cancer cell proliferation and stem cell differentiation, Research Number: 23781731, Research Results of Grants-in-Aid for Scientific Research (KAKENHI) published on May 15, 2013 and the like).

In a typical embodiment, the DNA aptamer of the present invention has a nucleotide sequence of SEQ ID NO: 1 or NO: 2 shown below. Furthermore, the nucleotide sequence is described from the left to the right starting from the 5' end to the 3' end.
<SEQ ID NO: 1> ACA GTT CGT CAG TGT TTG GGG TTC AGC TTA GGT G (34 mer)
<SEQ ID NO: 2> TGC GCG TGG GTG GTT TTT GTC TGT CAG CTT GGG TC (35 mer)

The DNA aptamer of the present invention may be the above-mentioned SEQ ID NO: 1 or 2 in which one or more of nucleotides are substituted, deleted, or added, as long as the DNA aptamer has the function to specifically bind to a non-small cell lung cancer cell. Preferably, the nucleotides that are substituted, deleted, or added are 1 to 3 nucleotide(s), more preferably 1 or 2 nucleotide(s), and further preferably 1 nucleotide. Moreover, when such substitution, deletion, or addition of a nucleotide is present, the sequence of the DNA aptamer of the present invention can be a sequence which shows a homology of 90% or more, preferably 93% or more, or more preferably 96% or more, to each of the above-mentioned SEQ ID NOs: 1 and 2 (hereinafter, they can be referred to as "homologs"). Here, when used in the present specification, the term "homology" is used with a meaning generally recognized in the present technical field. The term typically refers to the number of nucleotides of the nucleic acid sequence of the subject matter matching to the nucleotides identical to the reference nucleic acid sequence, when examined by a sequence analysis program (for example, Karlin and Altschul, 1990, PNAS 87:2264-2268; Karlin and Altschul, 1993, PNAS 90:5873-5877) or a visual inspection.

When one or more nucleotides are substituted, the substitution can be carried out by a universal base. The term "universal base" refers to a meaning that is generally recognized in the present technical field. The term, in general, refers to a nucleotide base analogue that forms a base pair with each base of a standard DNA/RNA without hardly any difference and which can be recognized by an intracellular enzyme (for example, Loakes et al., 1997, J. Mol. Bio. 270: 426-435). Non-limiting examples of universal bases include, C-phenyl, C-naphthyl, and other aromatic derivatives, inosine, azole carbozamide, and nitroazole derivative (3'-nitropyrole, 4-nitroindole, 5-nitroindole, 6-nitroindole, and the like) (Loakes, 2001, Nucleic Acids Res. 29: 2437).

Furthermore, there is no upper limit on the length of the DNA aptamer of the present invention as long as the DNA aptamer has the function to specifically bind to a non-small cell lung cancer cell. However, in view of the easiness of synthesis and problems of antigenicity and the like, the length of the DNA aptamer in the present embodiment, for example, as its upper limit, is 200 bases or less, preferably 150 bases or less, or more preferably 100 bases or less. When the number of the total bases is low, chemical synthesis and bulk production are easier, and there is a greater advantage in terms of cost. In addition, it is easily chemically modified, the safeness in the living body is high, and toxicity is low. The lower limit is provided as numbers that are same or more to the number of the bases in the above-mentioned SEQ ID NOs: 1 and 2, i.e., 34 bases or 35 bases or more. A DNA aptamer is preferably a single-stranded DNA (ssDNA); however, even in a case where a partial double-stranded structure is formed by adopting a hairpin loop type structure, the length of that DNA aptamer is calculated as a length of a single strand.

In a preferred embodiment, the DNA aptamer of the present invention is either one of the sequence of the above-mentioned SEQ ID NO: 1 or SEQ NO: 2, which can be a nucleotide sequence having a primer-primer recognition sequence at each 5' and 3' end. In other words, in this case, the DNA aptamer has the following nucleotide sequence:
5'-P₁-X-P₂-3'.
Wherein X is a nucleotide sequence selected from the sequences shown in SEQ ID NO: 1 and NO: 2 or is a sequence comprising 1 to 3 nucleotide substitutions, deletions, additions, in these sequences. P₁ and P₂ are a first and a second primer recognition sequence introduced for PCR amplification. Preferably, P₁ is GCC TGT TGT GAG CCT CCT (SEQ ID NO: 3) and P₂ is CGC TTA TTC TTG TCT CCC (SEQ ID NO: 4).

The DNA aptamer of the present invention may be chemically modified for an increase of the stability in the living body. Unlimited examples of such chemical modifications include a chemical substitution at the sugar chain moiety (for example, 2'-O methylation); a chemical substitution at the phosphate ester moiety (for example, phosphorothioation, amino group, lower alkyl amine group, acetyl group, and the like), and a chemical substitution at a base moiety. Similarly, an additional base could be provided at the 5' or 3' end. The length of such additional base is normally 5 bases or less. Additional bases may be a DNA or RNA; however, when DNA is used, the stability of the aptamer may increase. Sequences of such additional bases include sequences such as ug-3', uu-3', tg-3', tt-3', ggg-3', guuu-3', gttt-3', ttttt-3', and uuuuu-3', for example; however, they are not limited thereto.

Furthermore, for example, the DNA aptamer of the present invention can have a detection label linked to the 5' end or 3' end, for using it in the detection method of lung cancer cells or a kit used in the detection described hereinafter. As such detection label, fluorescent labelling is preferred; however, Raman label, enzyme label, and infrared label may be used. In fluorescent labelling, fluorescent label agent conventionally used in the present technical field could be used; however, for example, fluorophores that can be introduced by commercially available oligonucleotide solid phase synthesis services include such as 6-carboxytetramethylrhodamine (TAMRA (Trademark), fluorescein isothiocyanate (FITC), 6-carboxyfluorescein-aminohexyl (FAM), cyanine fluorescent dyes (Cy3, Cy5), and the like. Moreover, a quencher that absorbs a fluorescence energy emitted from the fluorescent substance may be further bound adjacent to a fluorescent substance. In such embodiment, fluorescence is detected by the fluorescent substance and quencher being separated at the time of detection reaction.

As examples of Raman labelling, an organic compound in particular a substance whose absorption band would, albeit only slightly, overlap with the laser wavelength is adsorbed onto a gold particle having a diameter of nanometer size, so that enhanced Raman scattering by using an enhanced electricity field formed by the surface plasma wave of the nanoparticle, could be used as a labelling agent. In such a case, fluorescent dyes are frequently used as an organic compound; however, this is not necessary a choice in particular, and dyes such as crystal violet can sufficiently obtain a Raman signal.
Examples of enzyme labelling include β-galactosidase, β-glucosidase, alkaliphosphatase, peroxidase, malate dehydrogenase, and the like. Moreover, as luminescent substrate, luminol, luminol derivative, luciferin, lucigenin, and the like may be used as a labelling agent.

### 2. Selection of DNA aptamers

The DNA aptamer of the present invention can be selected and obtained by using a well-known *in vitro* selection method in the present technical field. As preferable examples of such method, Systematic Evolution of Ligands by Exponential enrichment: SELEX method is used. SELEX method includes multiple repetition of selection of a nucleic acid ligand (aptamer) binding to a target substance and an exponential amplification by PCR, thereby a nucleic acid molecule (a single-stranded DNA, RNA) having an affinity to the target substance is obtained. Moreover, as its improved method, it is preferred to use Cell-SELEX method such as disclosed in Guo,et al.,Int.J.Mol.Sci.,9(4):668,2008, for example. As this method can use the cell itself as a target, in comparison to a conventional SELEX method, Cell-SELEX method has an advantage in that the analysis of the membrane protein of the cell surface is not required, a plural number of aptamers which can bind to the cell surface can be simultaneously selected, and aptamers more specifically binding to the target cell can be selected. Moreover, apart from using them, the DNA aptamer of the present invention can be selected and obtained using well-known methods in the preset technical field.

As mentioned above, *"in vitro* selection method" is a method which selects aptamer molecules having an affinity to the target molecules and cells from a nucleic acid molecule pool containing random nucleotide sequences (the so-called DNA pool) and removes molecules which do not have an affinity. Furthermore, this is a method that allows the enrichment of an aptamer molecule having a strong binding capacity by repeating the cycle which includes only amplifying the selected aptamer molecules using PCR methods and the like and performing the selection by affinity.

Specifically, first of all, a single-stranded nucleic acid molecule comprising a random nucleotide sequence (base sequence) region of approximately 20 to 300 bases, preferably, 30 to 150 bases, or more preferably 30 to 100 bases for example, an oligo DNA,, is prepared. It is preferred to use an oligo DNA having, a base sequence which serves as a primer to allow the PCR amplification at both ends. The primer recognition sequence moiety may have an appropriate restriction enzyme site so that the primer moiety could be excised by a restriction enzyme after a PCR amplification. The length of the primer recognition sequence moiety to be used is not particularly limited; however, it is about 20 to 50, preferably 20 to 30 bases. Moreover, for allowing the separation of a single-stranded DNA after PCR amplification by electrophoresis and the like, labelling such as with radioactive labelling and fluorescent labelling at the 5' end can be carried out.

Next, nucleic acid molecules having the random nucleotide sequence obtained as above (library pool) can be mixed with a target cell at an appropriate concentration ratio, and this is incubated under an appropriate condition. After incubation, the mixture is centrifuged to separate the nucleic acid molecule-target cell complex from free nucleic acid molecules. Then, the supernatant portion of the separated solution is removed and PCR reaction is carried out by using the obtained nucleic acid molecule-target cell complex to perform the amplification of the cell binding nucleic acid sequence. Subsequently, a nucleic acid molecule which had formed a complex with a target cell can be made into a single strand according to the well-known methods in the present technical field. Such means include the separation utilizing the binding of streptavidin-immobilized magnetic particles with biotin, for example. Due to this, ssDNA having a cell binding ability can be separated from an amplified nucleic acid double-stranded chain, and moreover, unnecessary coexisting substance contained in the PCR reaction solution such as DNA polymerase could be removed. After this, a similar operation is carried out using the collected ssDNA as a library pool.

A series of operations from mixing with the above-mentioned nucleic acid molecule and the target cell, separating the nucleic acid molecule bound with the target cell, PCR amplification, and using the amplified nucleic acid molecule once again in the binding with the target cell is carried out for a couple of rounds. By repeating the rounds, a nucleic acid molecule which more specifically binds with a target call can be selected. The sequence analysis of the obtained nucleic acid molecule can be carried out by the well-known methods in the present technical field.

### 3. Compositions for detecting cancer cells, Methods for detection, and Kit

As mentioned above, the DNA aptamer of the present invention has a function specifically binding to a non-small cell lung cancer cell, and thus, it can be preferably used in the detection of lung cancer cells. The composition for the detection comprising the DNA aptamer of the present invention can also be used as a tumor marker against non-small cell lung cancer.

Specifically, the composition for detection comprising a DNA aptamer of the present invention is contacted with samples obtained from the living body selected from the group consisting of lung cells, lung tissues, blood, blood serum, blood plasma, saliva, and sputum. Then, the detection of the presence of the lung cancer cell is made by observing the response (presence of the signal) caused by the binding of the sample and the DNA aptamer. "Samples obtained from the living body" are preferably obtained from animals, preferably from human; however, as long as the samples are samples or secreted body fluid that can be assured to be obtained at minimal invasion or *in vitro* cell culture solution component samples and the like, the form is not particularly restricted. Moreover, the "response" for detecting the presence of lung cancer cells is preferably a fluorescent response, and as mentioned above, it is preferred to link the fluorescent labelling agent such as TAMRA (Trademark) and FITC at the 5' or 3' end of the DNA aptamer.

The composition for detecting lung cancer cells of the present invention can be provided as a kit comprising a DNA aptamer enhancing the convenience or portability. In the kit, the DNA aptamer can be provided in an embodiment of an aqueous solution in which the DNA aptamer is generally dissolved at an appropriate concentration or in an embodiment of a DNA array in which the DNA aptamer is immobilized on a solid phase support. For example, biotin can be bound to the end of the DNA aptamer to form a complex, streptavidin can be immobilized on the surface of a solid phase support, and the DNA aptamer can be immobilized on the surface of the solid phase by the interaction of biotin and streptavidin. The kit may appropriately contain other reagents and the like as necessary, for example, additives such as solubilizing agents, pH adjusting agents, buffer agents, and tonicity agents can be used, and the formulation amount can appropriately be selected by those skilled in the art.

### 4. Pharmaceutical composition and DDS

In another embodiment, the present invention provides a pharmaceutical composition for preventing metastasis of or treating lung cancer, comprising the above-mentioned DNA aptamer. Preferably, in addition to the DNA aptamer, the pharmaceutical composition can comprise an effective amount of a pharmaceutical compound for preventing metastasis of or treating lung cancer (an active ingredient) and a pharmaceutically acceptable carrier. For example, those in which the present aptamer is bound to a gold nanoparticle may be utilized as a reagent in hyperthermia therapy for cancer.

The above-mentioned "lung cancer" is a non-small cell lung cancer comprising squamous cell carcinoma (ASC), adenocarcinoma (ADC), and large cell carcinoma (LCC), and preferably, it is an adenocarcinoma associated to H1975 cells. The phrase "preventing metastasis or treating" may include the suppression of liberation, migration, metastasis, infiltration, or proliferation of cancer cells and induction of apoptosis. The suppression of metastasis refers to suppressing the cancer cells arriving to a different site than the primary lesion and developing secondary cancer at the site.

The active ingredient contained in the pharmaceutical composition of the present invention is not particularly limited as long as it is effective for preventing metastasis of or treating lung cancer; however, it is preferably an anti-cancer agent. Examples of such anti-cancer agents include such as alkylating agents, anti-metabolic agents, antitumor antibiotics, chemotherapeutic agents, and anti-cancer agents other than those. Alkylating agents include, for example, nitrogen mustard, chlorambucil, dibromodulcitol, thiotepa, carmustine, busulfan, and the like. Anti-metabolic agents include 6-mercaptopurine, fluorouracil, tegafur, doxifluridine, cytarabine, enocitabine, methotrexate, and the like. Antibiotics include mitomycin C, bleomycin, peplomycin, doxorubicin, THP-adriamycin, actinomycin D, and the like. Other anti-cancer agents include amrubicin hydrochloride, irinotecan hydrochloride, ifosfamide, etoposidelastet, gefinitib, cyclophosphamide, cisplatin, trastuzumab, fluorouracil, imatinib mesylate, methotrexate, rituxan, adriamycin, carboplatin, tamoxifen, camptothecin, melphalan, L-asparaginase, aceclatone, sizofiran, and the like.

As a pharmaceutically acceptable carrier contained in the pharmaceutical composition of the present invention, for example, the following is included, but they are not restricted thereto: excipients such as sucrose and starch, binding agents such as cellulose and methylcellulose, disintegrating agents such as starch and carboxymethyl cellulose; lubricants such as magnesium stearate and aerosil; aromatic agents such as citric acid and menthol; preservatives such as sodium benzoate and sodium hydrogen sulfite; stabililzers such as citric acid and sodium citrate; suspending agents such as methyl cellulose and polyvinylpyrrolidone; dispersant such as surfactants; diluents such as water and saline, and base wax.

For promoting the introduction of the pharmaceutical composition of the present invention in the cancer cells, the composition may further comprise a nucleic acid introducing reagent. As such nucleic acid introducing reagent, atelocollagen, liposomes, nanoparticles, cationic lipids such as lipofectin, lipofectamine, DOGS (transfectum), DOPE, DOTAP, DDAB, DHDEAB, HDEAB, polybrene, or polyethylene imine, can be used.

The pharmaceutical composition of the present invention can be administered to mammals (e.g., human, rat, mouse, rabbit, sheep, pig, cow, cat, dog, monkey, and the like).

The pharmaceutical composition of the present invention can be made into a large variety of formulation forms, for example, in a dosage form of capsules, tablets, and liquid agents. Although without being restricted, in general, it is made into a liquid to be made into an injection, or into an oral agent or a sustained release agent. The injection could be prepared by well-known methods in the present technical field. For example, the injection could be prepared by dissolving the pharmaceutical composition of the present application in an appropriate solvent such as sterilized water, buffer solution, and saline, filtering this through a filter and the like for sterilization followed by filling into a sterile container. Furthermore, as an oral agent, for example, the pharmaceutical composition of the present application could be formulated into a dosage form of tablets, granules, fine granules, powders, soft or hard capsules, liquid agents, emulsions, suspending agents, and syrups. As a sustained-release agent, for example, the pharmaceutical composition of the present application could be formulated into a dosage form of tablets, granules, fine granules, powders, soft or hard capsules, and microcapsules. At formulation, preferably, for example, stabilizers such as albumin, globulin, gelatin, mannitol, glucose, dextran, and ethylene glycol can be added. Moreover, in the formulation of the pharmaceutical composition of the present invention, for example, necessary additives such as excipients, solubilizing agents, anti-oxidants, soothing agents, and isotonic agents may be included. Furthermore, when made into a liquid formulation, it is desirable that it is preserved by cryopreservation, freeze drying, or the like, where water is removed. The freeze dry agent is used with the addition of distilled water for injection and the like to be redissolved at the time when used. Moreover, when made into a sustained-release agent, as a carrier for sustained-release, for example, soluble collagens or derivatives of soluble collagen, proteins such as gelatins, porous ceramics, polyamino acids, polylactic acids, chitin or chitin derivatives, water-absorbing polymer gel, can be used.

The pharmaceutical composition of the present invention may be administered by an appropriate administration route depending on its form. The administration can be carried out orally or parentally; however, parental administration is desired. For example, the pharmaceutical composition can be administered in a form of injection by intravenous administration, arterial administration, subcutaneous administration, intramuscular injection, and the like. Furthermore, the pharmaceutical composition of the present application can be made into a sustained-release form for administration by embedding into the living body, for example, to the affected site, under the skin, or in the muscle. The amount of dose, the number of dose, and the like would be different depending on the purpose of administration, method of administration, kind and size of cancer, condition of the subject of administration (sex, age, weight, and the like); however, basically the administration is carried out according to the desirable administration form of the above-mentioned active ingredients.

Moreover, the DNA aptamer of the present invention can be attached to the surface of the transport material of liposomes, nanoparticles, and the like so that the pharmaceutical component contained in the transport material can be selectively transported to lung cancer cells. Therefore, in a further embodiment, the present invention provides a drug delivery system for preventing metastasis of or treating lung cancer, in which the drug delivery system contains the above-mentioned DNA aptamer. The pharmaceutical component that can be transported by the drug delivery system is typically the above-mentioned anti-cancer agent; however, as long as the pharmaceutical component is a useful substance for preventing metastasis of or treating lung cancer, in addition to the above-mentioned anti-cancer agents, it can be toxin, cancer growth inhibition gene, apoptosis gene, or an siRNA (small interfering RNA) and the like which inhibits the expression of an gene playing an important role in the growth and metastasis of lung cancer.

### EXAMPLES

Hereinafter, the present invention will be further described in detail with reference to the Examples, but the present invention is not limited thereto.

### Example 1: Selection of the aptamers

The selection of aptamers specifically binding to H1975 cells which are non-small cell lung cancer cells from DNA pools having random sequences has been carried out using Cell-SELEX method. The steps in the Cell-SELEX method are as follows:
1) Preparation of DNA pools (preparation of the solution of a group of DNA aptamer candidates)
2) Mixture of the target substance and H1975 cells
3) Separation of target binding DNA and non-binding DNA
4) Duplication of the target binding DNA (a step of amplifying the DNA aptamer bound to the target substance)
5) Purification of target binding DNA (a step of purifying the amplified DNA aptamer to a single-stranded DNA)
6) Cloning of the target binding nucleic acid (pretreatment of the sequence analysis of the obtained DNA aptamer)
7) Performing 8 rounds of the steps of these 1) to 6)
8) Sequence analysis of the target binding nucleic acid (Analysis of the nucleotide sequence of the DNA aptamer using a sequencer)

Hereinbelow, a further experimental procedure in details is provided.

For a DNA pool, an oligo DNA having the following sequence whose full length is 70 bases and the random sequence portion (N) is 34 bases is used.

DNA pool: Random34 (Manufacture by Tsukba Oligo Service Co., Ltd)
- Sequence: 5'-GCC TGT TGT GAG CCT CCT(N₃₄)CGC TTA TTC TTG TCT CCC-3'
- Length: 70 bases (the random sequence is of the 34 bases in the middle)
- Molecular weight: 21391.3 g/mol
- Molar absorption coefficient: 630475 L/mol·cm

Both ends of the random sequence are sequences that are recognized by a primer in the subsequent PCR and that allow the amplification. The above-mentioned random DNA was made into to a 1 υM DNA pool using a buffer of a cell culture medium (Wako pure chemical industries Ltd: RPM1-1640 with glucose + bovine calf serum + antibiotics) as a solvent.

Then, H1975 cells were cultured at a culture dish until the number of the cells would become 10⁶ to 10⁷. Subsequently, after culturing, the culture medium was removed and to the same dish, 2 mL of phosphate buffered saline (hereinafter, referred to as PBS) was added and the cells were washed. To this plate, 1 mL of a 0.05% EDTA containing trypsin solution was added and the plate was allowed to stand still for 2 minutes at 37°C in an incubator. Once the cells were confirmed to be coming off from the plate, 4 mL of PBS was added, and this was collected in a 15 mL centrifuge tube to be centrifuged at 200 g for 3 minutes. Then, the supernatant solution was removed by an aspirator. To this, 3 mL of PBS was added and the cells were suspended by pipetting followed by centrifugation at 200 g for 3 minutes, and the washing step of removing the supernatant was carried out twice. The culture medium was prepared so that it would be 10⁶ cells in 330 uL. The prepared suspension solution was mixed with 370 uL of 1 uM of random DNA (DNA pool) solution previously prepared, and this was sufficiently mixed by vortexing.

The obtained mixed solution was stand still at 37°C for 1 hour in an incubator. Then, the same centrifuge tube was used to be centrifuged at 400 g for 4 minutes, and then the supernatant was removed. To this, 500 υl of PBS was added and centrifugation was carried out at 400 g for 4 minutes. This washing operation was carried out 3 times. Once the supernatant was removed, 200 υL of PBS was added and heated at 95°C for 10 minutes. Centrifugation was carried out at 13000 g for 5 minutes and the supernatant was collected.

The H1975 cell binding DNA which was separated was amplified by PCR. As for the device, Thermal Cycler (TAKARA-TP600) was used. As for the primers, a primer of 18 bases corresponding to the common sequence of the above-mentioned random DNA was used (Manufactured by Tsukuba Oligo Service Co., Ltd). A biotin modification at the 5' terminal side of the primer is carried out to allow the separation of the single-stranded DNA as mentioned below.

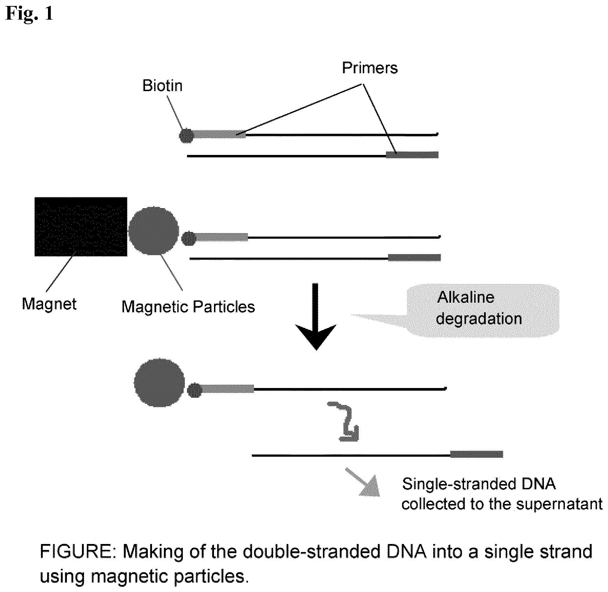

Streptavidin was added to the purified DNA to be adsorbed to magnetic particles and the supernatant was removed once the magnetic particles were recovered using magnets, and then a single-stranded DNA which did not bind to the magnetic particles was collected in the supernatant by alkaline buffer degradation (Fig. 1). Subsequently, alkaline buffer was substituted with a PBS buffer and the target single-stranded DNA binding to a magnetic particle was collected. This procedure was considered as one round, and this operation was carried out 8 times.

Once the 8 rounds were completed, PCR amplification was performed using a non-biotinylated primer of 18 bases, and the PCR product was analyzed using a sequencer. The analysis was carried out using a sequence analysis device Ion PGMTM (Registered Trademark) manufactured by Life Technologies Japan Ltd., and then, by using the method similar to that of the above-mentioned, a DNA aptamer targeting each of the mouse whole blood and mouse mononuclear cells is independently screened by Cell-SELEX method, and those having a common sequence with the DNA aptamer sequences targeting mouse whole blood and mouse mononuclear cells were discarded from a group of the already found DNA aptamers against H1975 cells. As a result of this, Sequence 1 and Sequence 2 were found as sequences only binding to H1975 cells but not binding to mouse whole blood and mouse mononuclear cells.

### Example 2: Staining of a non-small cell lung cancer cell using a fluorescent labeled DNA aptamer

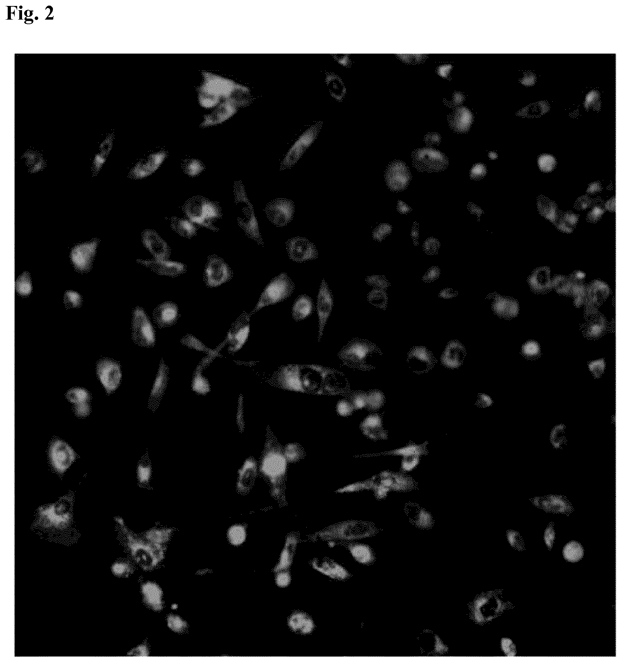

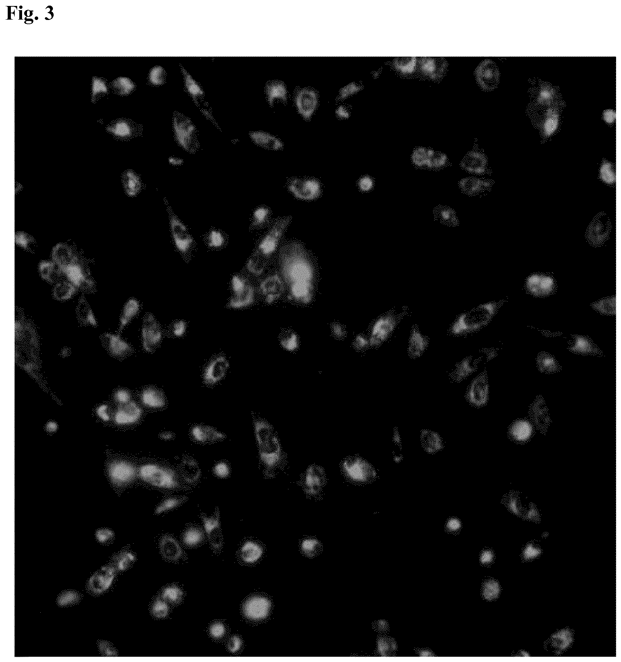

H1975 cells were cultured in a culture dish until the cell number reached to 10⁶ to 10⁷. The 5' end of a DNA aptamer consisting of a nucleotide sequence having an affinity to the H1975 cells found by sequencing analysis was modified with Cy3.5 (Trademark), 30 ul of the aptamer solution in which the DNA was adjusted to be 100 uM with ultrapure water was added to the culture dish with dispersing (culture medium RPMI-1640; 2mL). This was allowed to stand in an incubator at 37°C for 1 hour. After this, the cells were washed 3 times with PBS, and then they were observed under the inverted fluorescence microscope IX51 manufactured by Olympus at a condition where 2 mL of PBS was added. The results by using the DNA aptamers having SEQ ID NOs: 1 and 2 are each shown in Fig. 2 and Fig. 3. Accordingly, it was revealed that these fluorescent labelled DNA aptamers specifically bind to H1975 cells and a well fluorescence image for H1975 cells were obtained.

## Claims

1. A DNA aptamer having at least one nucleotide sequence selected from the sequence of SEQ ID NOs: 1 and 2 and **characterized by** specifically binding to a non-small cell lung cancer cell.

2. A DNA aptamer having a sequence comprising substitutions, deletions, or additions of 1 to 3 nucleotides, in at least one nucleotide sequence selected from the sequence of SEQ ID NOs: 1 and 2 and **characterized by** specifically binding to a non-small cell lung cancer cell.

3. A DNA aptamer specifically binding to a non-small cell lung cancer cell wherein the DNA aptamer has a nucleotide sequence set forth as 5'-P₁-X-P₂-3', wherein X is 1) a nucleotide sequence selected from the sequences of SEQ ID NOs: 1 and 2, or 2) a sequence comprising substitutions, deletions, or additions 1 of 1 to 3 nucleotides, in a nucleotide sequence selected from the sequences of SEQ ID NOs: 1 and 2, and
P₁ and P₂ are a first and a second primer recognition sequence introduced for PCR amplification.

4. The DNA aptamer according to claim 3, wherein P₁ is a first primer recognition sequence of SEQ ID NO: 3 and P₂ is a second primer recognition sequence of SEQ ID NO: 4.

5. The DNA aptamer according to any one of claims 1 to 4, wherein the non-small cell lung cancer cell is a H1975 cell.

6. The DNA aptamer according to any one of claims 1 to 5, comprising at least one chemical modification selected from the group consisting of a chemical substitution at the sugar chain moiety, a chemical substitution at the phosphate ester moiety, and a chemical substitution at the base moiety of nucleic acid.

7. The DNA aptamer according to any one of claims 1 to 6, having a fluorescent label at the 5' or 3' end.

8. The DNA aptamer according to claim 7, wherein the fluorescent label is 6-carboxy tetramethyl rhodamine, fluorescein isothiocyanate, 6-carboxy fluorescein-aminohexyl, or a cyanine family fluorescent dye.

9. A composition for detecting lung cancer cell, comprising the DNA aptamer according to any one of claims 1 to 8.

10. A kit for detecting lung cancer cell, comprising the DNA aptamer according to any one of claim 1 to 8.

11. A method for detecting lung cancer, **characterized by** using the DNA aptamer according to any one of claims 1 to 8.

12. The method according to claim 11, comprising a step of contacting the DNA aptamer with a sample obtained from a living body selected from the group consisting of lung cells, lung tissues, blood, blood serum, blood plasma, saliva, and sputum and a step of detecting the presence of a lung cancer cell by observing the response caused by the binding of the sample and the DNA aptamer.

13. The detection method according to claim 12, wherein the response is a fluorescent response or a Raman scattering response.

14. A pharmaceutical composition for preventing metastasis of or treating lung cancer, comprising the DNA aptamer according to any one of claims 1 to 8.

15. A use of the DNA aptamer according to any one of claims 1 to 8 for the production of a pharmaceutical composition for preventing metastasis of or treating lung cancer.

16. A drug delivery system for preventing metastasis of or treating lung cancer, comprising the DNA aptamer according to any one of claims 1 to 8.


### SEQUENCE LISTING

SEQUENCE LISTING

<110>  Nissan Chemical Industries,Ltd.

<120>  Aptamer that binds to a lung cancer cell H1975

<130>  10092

<160>  5

<170>  PatentIn version 3.5

<210>  1
<211>  34
<212>  DNA
<213>  Artificial Sequence

<220>
<223>  DNA Aptamer that binds to lung cancer cell H1975

<400>  1
acagttcgtc agtgtttggg gttcagctta ggtg                                    34


<210>  2
<211>  35
<212>  DNA
<213>  Artificial Sequence

<220>
<223>  DNA Aptamer that binds to lung cancer cell H1975

<400>  2
tgcgcgtggg tggtttttgt ctgtcagctt gggtc                                   35


<210>  3
<211>  18
<212>  DNA
<213>  Artificial Sequence

<220>
<223>  Primer binding to site P1

<400>  3
gcctgttgtg agcctcct                                                      18


<210>  4
<211>  18
<212>  DNA
<213>  Artificial Sequence

<220>
<223>  Primer binding to site P2

<400>  4
cgcttattct tgtctccc                                                      18


<210>  5
<211>  70
<212>  DNA

<213>  Artificial Sequence

<220>
<223>  DNA Pool random 34



<220>
<221>  misc_feature
<222>  (19)..(52)
<223>  n is a, c, g, or t

<400>  5
gcctgttgtg agcctcctnn nnnnnnnnnn nnnnnnnnnn nnnnnnnnnn nncgcttatt        60

cttgtctccc                                                                70